# EUROPEAN PATENT SPECIFICATION

(11) **EP 0 785 794 B1**
(45) Date of publication and mention of the grant of the patent: **19.12.2001**
(21) Application number: 95935725.2
(22) Date of filing: 13.10.1995
(51) Int. Cl.: A61K 31/551, A61K 9/28

(54) **ENTERIC COATED COMPOSITIONS OF 1,5-BENZODIAZEPINE DERIVATIVES HAVING CCK ANTAGONISTIC OR AGONISTIC ACTIVITY**
ZUBEREITUNGEN VON 1,5-BENZODIAZEPINDERIVATEN MIT CCK-ANTAGONISTISCHEN ODER AGONISTISCHER WIRKUNG MIT ENTERISCHEM ÜBERZUG
COMPOSITIONS GASTRO-RESISTANTES A BASE DE DERIVES DE BENZODIAZEPINE-1,5 PRESENTANT UNE ACTIVITE CCK ANTAGONISTE OU AGONISTE

(30) Priority: 14.10.1994 GB 9420748
(43) Date of publication of application: 30.07.1997
(73) Proprietor: GLAXO WELLCOME INC., Research Triangle Park, North Carolina 27709 (US)
(72) Inventor: SUGG, Elizabeth, Ellen, Research Triangle Park, NC 27709 (US)
(74) Representative: Learoyd, Stephanie Anne
(86) International application number: US9512829
(87) International publication number: WO9611701

(56) References cited:
- WO-A-90/06937
- WO-A-93/14074
- WO-A-94/24149

## Description

This invention relates to pharmaceutical compositions containing novel 1,5-benzodiazepine derivatives which exhibit agonist activity for CCK-A receptors thereby enabling them to modulate the hormones gastrin and cholecystokinin (CCK) in mammals.

Cholecystokinins (CCK) and gastrin are structurally related peptides which exist in gastrointestinal tissue and in the central nervous system. Cholecystokinins include CCK-33, a neuropeptide of thirty-three amino acids in its originally isolated form, its carboxyl terminal octapeptide, CCK-8 (also a naturally occurring neuropeptide), and 39- and 12-amino acid forms. Gastrin occurs in 34-, 17- and 14- amino acid forms, with the minimum active sequence being the C-terminal tetrapeptide, Trp-Met-Asp-Phe-NH₂ (CCK-4) which is the common structural element shared by both CCK and gastrin.

CCK and gastrin are gastrointestinal hormones and neurotransmitters in the neural and peripheral systems and perform their respective biological roles by binding to particular receptors located at various sites throughout the body. There are at least two subtypes of cholecystokinin receptors termed CCK-A and CCK-B and both are found in the periphery and in the central nervous system.

The CCK-A receptor, commonly referred to as the "peripheral-type" receptor, is primarily found in the pancreas, gallbladder, ileum, pyloric sphincter and on vagal afferent nerve fibers. Type-A CCK receptors are also found in the brain in discrete regions and serve to provide a number of CNS effects. Due to the ability of CCK-8 and Type-A CCK-selective agonists to suppress food intake in several animal species, considerable interest has been generated toward the development of new substances which function as Type-A receptor-selective CCK agonists in order to serve as anorectic agents.

The CCK-B or gastrin receptors are found in peripheral neurons, gastrointestinal smooth muscle and gastrointestinal mucosa, most notably in parietal cells, ECL cells, D cells and chief cells. CCK-B receptors also predominate in the brain and have been implicated in the regulation of anxiety, arousal and the action of neuroleptic agents.

U.S. Patent No. 4,988,692, to Gasc, et al. describes a group of 3-acylamino 1-alkyl-5-phenyl 1,5-benzodiazepine derivatives which behave as cholecystokinin antagonists to reverse or block the effects of the endogenous hormone at its receptors.

US Patent No. 4,490,304 and PTC applications No's WO90/06937 and WO91/19733 describe peptide derivatives that exhibit CCK-A agonist activity. Such compounds have been disclosed for appetite regulation as well as the treatment and/or prevention of gastrointestinal disorders or disorders of the central nervous in animals and, more particularly, humans.

We have now discovered that the bioavailability following oral administration of a novel group of 3-amino 1,5-benzodiazepine compounds which exhibit a agonist activity for the CCK-A receptor may be significantly increased if the compound is administered in a solid dosage form the outer layer of which is an enteric coating or shell.

The present invention thus provides a pharmaceutical formulation in solid dosage form for oral administration which comprises a compound of the general Formula (I) 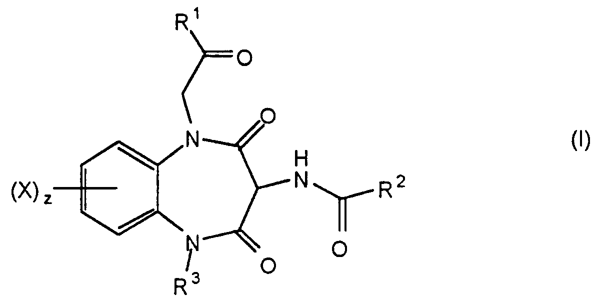 and physiologically salts and solvate thereof wherein:
X is either hydrogen, trifluoromethyl, alkyl, C₁₋₄alkylthio, -O(C₁₋₄alkyl) or halogen;
R¹ is either Formula II or -NR⁴R⁵; 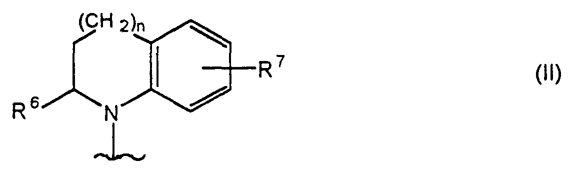
R² is either:
   (1) a heterocycle linked at its 2- position and selected from pyrrole, tetrahydropyrrole, indole, benzofuran, thiophene, benzothiophene, indoline, quinoline or 4-oxobenzopyran and wherein said pyrrole, tetrahydropyrrole, indole or indoline may optionally be substituted on the ring nitrogen thereof by the group R⁸ as defined hereunder and said indole, indoline, quinoline, benzofuran, benzothiophene or 4-oxo-benzopyran may optionally be substituted in the benzo ring thereof by the group R⁹ as defined hereunder or
   (2) phenyl or phenyl mono- or disubstituted independently with halogen, hydroxy, cyano, carboxy, -O(C₁₋₄alkyl), -O(CH₂C₆H₅), -COO(C₁₋₄alkyl), amino, dimethylamino, -NHR¹⁰, 1-pyrrolidinyl or tetrazolyl; or
   (3) pyridine or pyridinyl mono- or disubstituted independently with halogen, methyl, hydroxy, nitro, cyano, carboxy, -O(C₁₋₄ alkyl), -O(CH₂C₆H₅), -COO(C₁₋₄alkyl), amino or dimethylamino; or
   (4) -NHR¹¹ where R¹¹ is defined hereinunder or R¹¹ is 7-indazolyl containing a group R¹⁰ at the N-1 position;
R³ is hydrogen, C₁₋₆alkyl, C₃₋₆cycloalkyl, phenyl or phenyl mono- or disubstituted independently with halogen;
R⁴ is independently C₃₋₆alkyl, C₃₋₆cycloalkyl, C₃₋₆alkenyl, phenyl, -(CH₂)ₚCN or -(CH₂)ₚCOO(C₁₋₄alkyl) and R⁵ is independently C₃₋₆alkyl, C₃₋₆cycloalkyl, C₃₋₆ alkenyl, benzyl, phenyl or phenyl mono- or disubstituted independently with C₁₋₃alkyl optionally substituted with 1 or more fluorine atoms, cyano, hydroxy, dimethylamino, -O(C₁₋₄alkyl), -O(CH₂C₆H₅),-NH(C₁₋₄alkyl), -COO(C₁₋₄alkyl), -N(C₁₋₄alkyl)₂ pyrrolidino, morpholino or halogen or R⁴ is C₁₋₂alkyl and R⁵ is phenyl substituted at the 2- or 4-position with chloro, methyl, methoxy or methoxycarbonyl;
R⁶ is hydrogen or methyl;
R⁷ is hydrogen, hydroxy, fluoro, dimethylamino, -O(C₁₋₄alkyl) or -O(CH₂C₆H₅);
R⁸ is -(CH₂)_{b}COOH;
R⁹ is methyl, chloro, nitro, hydroxy, methoxy or -NHR¹⁰;
R¹⁰ is hydrogen, acetyl, C₁₋₄alkyl, -SO₃H, -SO₂CH₃, -SO₂CF₃ or -SO₂C₆H₅, C₁₋₄alkoxycarbonyl;
R¹¹ is phenyl or phenyl mono- or disubstituted independently with fluorine, trifluoromethoxy, C₁₋₄alkylthio, -(CH₂)_{c}COOH, -(CH₂)_{c}COO(C₁₋₄alkyl), -(CH₂)_{c}SCH₃, -(CH₂)_{c}SOCH₃, -(CH₂)_{c}SO₂CH₃, -(CH₂)_{c}CONH₂, -SCH₂COOH, -CONH(SO₂CH₃), -CONH(SO₂CF₃), -(CH₂)_{c}N(C₁₋₄alkyl)₂,-(CH₂)_{c}NH(SO₂CF₃), -(CH₂)_{c}N(SO₂CF₃)(C₁₋₄alkyl), -(CH₂)_{c}SO₂NHCO(C₁₋₄alkyl), -(CH₂)_{c}SO₂N(C₁₋₄alkyl)CO(C₁₋₄alkyl), -(CH₂)_{c}CONHSO₂(C₁₋₄alkyl), -(CH₂)_{c}CON(C₁₋₄alkyl)SO₂(C₁₋₄alkyl), -(CH₂)_{c}OR¹² -(CH₂)_{c}NHR¹⁰ or phenyl monosubstituted with -(CH₂)_{c}(tetrazolyl), -(CH₂)_{c} (carboxamidotetrazolyl) or -(CH₂)_{c}(pyrrolidinyl) or R¹¹ is selected from pyridine or pyridinyl mono- or disubstituted independently with halogen, methyl, hydroxy, nitro, cyano, carboxy, -O(C₁₋₄ alkyl), amino, dimethylamino, -NHR¹⁰;
R¹² is hydrogen, C₁₋₆alkyl, C₃₋₆cycloalkyl, -CH₂C₆H₅, -CH₂COOH, -CH₂CONH₂, -CH₂CONH(C₁₋₄alkyl), -CH₂CON(C₁₋₄alkyl)₂ or 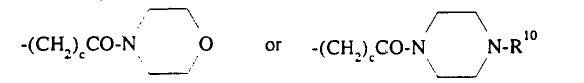
z is 1 or 2;
n is 1 or 2;
p is an integer from 1-4;
b is an integer from 0-3; and
c is 0 or 1.
together with one or more pharmaceutically acceptable carriers, wherein the formulation is encased in an enteric coating or capsule.

Conveniently the formulation according to the invention is in the form of a tablet coated with a conventional enteric coating. Alternatively the formulation according to the invention may be presented in the form of a capsule the shell of which is made from enteric material or is coated with an enteric material.

In the context of this application it will be understood that the term enteric coating or material refers to a coating or material that will pass through the stomach essentially intact but will rapidly disintegrate in the small intestine to release the active drug substance.

The tablets and capsules for oral administration may contain conventional excipients such as binding agents, (for example, syrup, acacia, gelatin, sorbitol, tragacanth, mucilage of starch or polyvinylpyrrolidone), fillers (for example, lactose, sugar, microcrystalline cellulose, maize-starch, calcium phosphate or sorbitol), lubricants (for example, magnesium stearate, stearic acid, talc polyethylene glycol or silica), disintegrants (for example, potato starch or sodium starch glycollate) or wetting agents, such as sodium lauryl sulphate. The enteric coatings may be applied to the tablets and/or capsules according to methods well-known in the art.

Suitable enteric coatings for use in the invention will be these coatings known to those skilled in the art. Such coatings include cellulose acetate phthalate, polyvinyl acetate phthalate, shellac, styrene maleic acid copolymers, methyacrylic acid copolymers and hydroxypropyl methyl cellulose phthalate. The said coatings may also contain a plasticizer and or a dye.

In the compounds of formula (I) when R¹ represents the group of Formula (II), examples of such a group include those wherein R⁶ is hydrogen or more particularly methyl, R⁷ is hydrogen, hydroxyl, methoxy, or fluorine, and n is 1.
In the compounds of formula I when R¹ represents the group of Formula (II), examples of such a group include those wherein R⁶ is hydrogen or more particularly methyl, R⁷ is hydrogen, hydroxyl, methoxy, or fluorine, and n is 1.

When R¹ represents the group NR⁴R⁵ , examples of suitable groups include those wherein R⁴ represent C₃₋₆ alkyl, such as propyl or isopropyl, cyclohexyl or phenyl and R⁵ represents C₃₋₆ alkyl, benzyl or phenyl optionally substituted in the para- position by hydroxy, dimethylamino methoxy, trifluoromethyl, fluorine, pyrrolidino or morpholino. Within this group, particularly useful R¹ groups include those wherein R⁴ is propyl and, more particularly, isopropyl and R⁵ represents phenyl or phenyl substituted in the para-position by groups selected from hydroxy, methoxy dimethylamino, fluorine, or morpholino.

Examples of particularly suitable R¹ groups include those wherein R¹ is the group of Formula (II) wherein R₆ is methyl, n is 1 and R⁷ is hydrogen, hydroxy, fluorine or methoxy or R¹ is the group NR⁴R⁵ wherein R⁴ is propyl or isopropyl and R⁵ is phenyl optionally substituted in the para position by a group selected from hydroxy, methoxy, fluoro, dimethylamino, pyrrolidino or morpholino.

When R² represents a group selected from indole, indoline, benzofuran, benzothiophene, quinoline or 4-oxobenzopyran, the optional substituent R⁹ is conveniently a group selected from hydrogen, methyl, methoxy, hydroxy, nitro or amino and, where appropriate, the optional substituent on nitrogen, (R⁸), is -CH₂CO₂H.

When R² is an optionally substituted phenyl group, this is conveniently phenyl or phenyl substituted by one or two groups, which may be the same or different and selected from chlorine, fluorine, amino, hydroxy or carboxyl.

When R² represents the group NHR¹¹, R¹¹ is conveniently phenyl (optionally substituted by fluoro, hydroxy, amino, dimethylamino, trifluoromethylsulphonylamino, C₁₋₄ alkoxycarbonyl, carboxy, 1 H-tetrazol-5-yl, acetylamino or OR¹² wherein R¹² represents hydrogen, methyl, benzyl, CH₂CO₂H, CH₂CONH₂, CH₂CONHCH₃, CH₂CON(CH₃)₂ 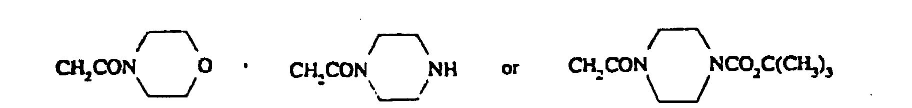 ) or a 7-indazolyl group wherein the N-1 substituent, (R¹⁰), is hydrogen.

When R¹¹ is a mono substituted phenyl group, the substituted is conveniently in the meta- position.

Examples of particularly suitable R² groups includes indole, benzofuran, thiophene, benzothiophene, indoline, quinoline, 4-oxobenzopyran, an optionally substituted phenyl group or the group NHR¹¹. Conveniently, R² is selected from the group indole, indoline or benzofuran, an optionally substituted phenyl group or the group NHR¹¹. More particularly, R² represents an indole, an optionally substituted phenyl or NHR¹¹.

When R³ represents C₁₋₆ alkyl, examples of suitable groups include methyl, ethyl, propyl, isopropyl, butyl, t-butyl or isoamyl.

When R³ represents C₃₋₆ cycloalkyl, examples of suitable groups include cyclopropyl, cyclopentyl or cyclohexyl.

When R³ represents phenyl, mono or disubstituted by independently with halogen, examples of suitable groups include those wherein the halogen substituent is fluorine e.g., 2-fluorophenyl or 4 fluorophenyl.

Examples of particularly suitable R³ groups include hydrogen, methyl, cyclohexyl, 2-fluorophenyl or phenyl, and more particularly, phenyl.

A particularly useful group of compounds for use in the formulations according to the invention include those wherein R¹ represents the group of Formula (II) wherein R⁶ is methyl, n is 1 and R⁷ is hydrogen, fluorine, hydroxy or methoxy, or more particularly NR⁴R⁵ wherein R⁴ is propyl or isopropyl and R⁵ is phenyl optionally substituted in the para position by a group selected from hydroxy, methoxy, fluoro, dimethylamino or morpholino; R² represents phenyl (optionally substituted independently by one or two groups selected from chlorine, fluorine, hydroxy, amine or carboxy), NHR¹¹ wherein R¹¹ represents phenyl (optionally substituted by amino, dimethylamino, trifluoromethyl- sulphonylamino, carboxy, 1H-tetrazol-5-yl, acetylamino or OR¹² wherein R¹² represents hydrogen, methyl, benzyl, CH₂CO₂H, CH₂CONH₂, CH₂CONHCH₃, CH₂CON(CH₃)₂, 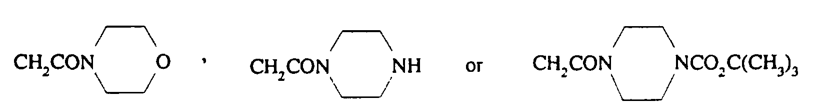 and wherein the substituent is preferably in the meta- position) or an indole wherein the nitrogen atom is optionally subtituted by the group -CH₂CO₂H and the benzo ring is optionally substituted by chlorine, methyl, methoxy, nitro, hydroxy or amino; R³ represents hydrogen, methyl, cyclohexyl, 2-fluorophenyl or phenyl or, more particularly, 2 fluorophenyl or phenyl; and X represents fluorine and z is 1 or, more particularly, X is hydrogen;

A particularly interesting class of compounds for use in the formulation according to the present invention because they exhibit a very high and selective affinity for the CCK-A receptor as well as exceptional efficacy are those wherein R² is an indole group. A preferred group of compounds within this class are those wherein the indole group is substituted on the nitrogen atom by the group -CH₂CO₂H or, more preferably, the nitrogen atom is unsubstituted, and benzo ring of the indole group is optionally substituted by a group selected from chlorine, methyl, methoxy, nitro, hydroxy or amino.

A particularly preferred compound for use in the formulation according to the invention is :
1 H-lndole-2-carboxylic acid {1-[Isopropyl-(4-methoxyphenyl)carbamoylmethyl]-2,4-dioxo-5-phenyl-2,3,4,5-tetrahydro-1H-benzo[b][1,4]diazepin-3-yl}amide and enantiomers thereof.

As provided herein, the term alkyl is generally intended to mean both straight chain and branched chain aliphatic isomers of the corresponding alkyl. For example, C₁₋₆alkyl is intended to include methyl, ethyl, n-propyl, isopropyl, n-butyl, isobutyl, tertbutyl, n-pentyl, etc.

The term cycloalkyl, as provided herein, is intended to mean all alicyclic isomers of the corresponding alkyl. For example, the term C₃₋₆ alkyl, as provided herein, is intended to include such groups as cyclopropyl, cyclopentyl and cyclohexyl.

The term halogen is intended to mean F, Cl, Br or I.

The term tetrazole as a group or part of a group refers to the (1 H)-tetrazol-5-yl grouping and tautomers thereof.

Those skilled in the art will recognize that stereocenters exist in compounds of Formula (I). Accordingly, the present invention includes all possible stereoisomers and geometric isomers of Formula (I) and includes not only racemic compounds but also the optically active isomers as well. When a compound of Formula (I) is desired as a single enantiomer, it may be obtained either by resolution of the final product or by stereospecific synthesis from either isomerically pure starting material or any convenient intermediate. Resolution of the final product, an intermediate or a starting material may be effected by any suitable method known in the art. See, for example, Stereochemistry of Carbon Compounds by E. L. Eliel (Mcgraw Hill, 1962) and Tables of Resolving Agents by S. H. Wilen. Additionally, in situations where tautomers of the compounds of Formula (I) are possible, the present invention is intended to include all tautomeric forms of the compounds.

It will also be appreciated by those skilled in the art that the compounds of the present invention may also be utilized in the form of a pharmaceutically acceptable salt or solvate thereof. The physiologically acceptable salts of the compounds of Formula (I) include conventional salts formed from pharmaceutically acceptable inorganic or organic acids as well as quaternary ammonium acid addition salts. More specific examples of suitable salts include hydrochloric, hydrobromic, sulphuric, phosphoric, nitric, perchloric, fumaric, acetic, propionic, succinic, glycolic, formic, lactic, maleic, tartaric, citric, pamoic, malonic, hydroxymaleic, phenylacetic, glutamic, benzoic, salicylic, fumaric, toluenesulphonic, methanesulphonic, naphthalene-2-sulphonic, benzenesulphonic and the like. Other acids such as oxalic, while not in themselves pharmaceutically acceptable, may be useful in the preparation of salts useful as intermediates in obtaining the compounds of the invention and their pharmaceutically acceptable salts. References hereinafter to a compound for use in the invention include both compounds of Formula (I) and their pharmaceutically acceptable salts and solvates.

The compounds of formula (I) exhibit CCK-A agonist activity and can be considered full or partial cholecystokinin agonists in that they bind to CCK-A receptors and either fully or partially stimulate gallbladder contraction and/or reduce feeding in animal paradigms.

As agonists of CCK-A receptors, the compounds of formula (I) are useful anorectic agents advantageous in the treatment of obesity as well as related pathologies, such as diabetes or hypertension. Moreover, the compounds disclosed herein provide for new approaches for inducing satiety, providing for appetite regulation and modifying food intake in mammals, especially humans, to regulate appetite, treat obesity and maintain weight loss.

Additionally, certain compounds of formula (I) may also exhibit some antagonist activity at particular site-specific CCK-B and gastrin receptors as demonstrated by their inhibition of CCK-4 stimulated contraction of isolated guinea-pig ileum longitudinal muscle-myenteric plexus and pentagastrin-stimulated acid secretion in rat isolated gastric mucosa using the procedures described by M. Patel and C. F. Spraggs in Br. J. Pharmac., (1992), 106, 275-282 and by J. J. Reeves and R. Stables in Br. J. Pharmac., (1985), 86, 677-684.

The relative affinities of compounds of formula (I) for the CCK-A and CCK-B receptors may be determined using known conventional procedures such as described by Fornos et al J. Pharmacol Exp. Ther., 1992 261, 1056-1063.

The ability of compouds of formula (I) to inhibit gastric acid secretion, such as pentagastrin stimulated acid secretion may be determined in the conscrious gastric fistula rat using methods described by Hedges and Parsons Journal of Physiology 1977, 267 191-194.

According to a further aspect of the present invention, there is provided herein a method for the treatment of a mammal, including man, in particular in the treatment conditions where modification of the effects of CCK and/or gastrin is of therapeutic benefit, the method comprising administering to the patient an enterically coated tablet or capsule containing a therapeutically effective amount of a compound of Formula (I) or a pharmaceutically acceptable salt or solvate thereof.

It will be appreciated by those skilled in the art that reference herein to treatment extends to prophylaxis as well as the treatment of established diseases or symptoms. Moreover, it will be appreciated that the amount of a compound of the invention required for use in treatment will vary with the nature of the condition being treated and the age and the condition of the patient and will be ultimately at the discretion of the attendant physician or veterinarian. In general, however, doses employed for adult human treatment will typically be in the range of 0.02 - 5000 mg per day, e.g., 1-1500 mg per day. The desired dose may conveniently be presented in a single dose or as divided doses administered at appropriate intervals, for example as two, three, four or more sub-doses per day.

Compounds of general Formula (I), methods for their preparation and their activity as CCK-A agonists are described in W09424149.

### Example

The following example illustrates the invention but should not be construed as a limitation thereto.

| **Tablet** | |
|---|---|
| Active Ingredient | 50 mg |
| Lactose anhydrous USP | 163 mg |
| Microcrystalline Cellulose NF | 69 mg |
| Pregelatinized starch Ph. Eur. | 15 mg |
| Magnesium stearate USP | 3 mg |
| Compression weight | 300 mg |

The active ingredient, microcrystalline cellulose, lactose and pregletinized starch are sieved through a 500 micron sieve and blended in a suitable mixer. The magnesium starate is sieved through a 250 micron sieve and blended with the active blend. The blend is compressed into tablets using suitable punches, then coated with cellulose acetate phthalate in a conventional manner.

## Claims

1. A pharmaceutical formulation in solid dosage form for oral administration which comprises a compound of Formula (1) 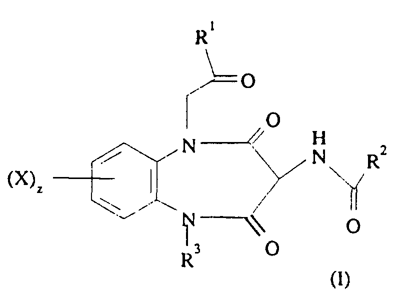 and physiologically salts and solvate thereof wherein:
X is either hydrogen, trifluoromethyl, alkyl, C₁₋₄alkylthio, -O(C₁₋₄alkyl) or halogen;
R¹ is either Formula II or -NR⁴R⁵; 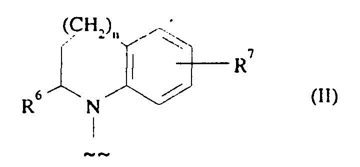
R² is either:
(1) a heterocycle linked at its 2- position and selected from pyrrole, tetrahydropyrrole, indole, benzofuran, thiophene, benzothiophene, indoline, quinoline or 4-oxobenzopyran and wherein said pyrrole, tetrahydropyrrole, indole or indoline may optionally be substituted on the ring nitrogen thereof by the group R⁸ as defined hereunder and said indole, indoline, quinoline, benzofuran, benzothiophene or 4-oxo-benzopyran may optionally be substituted in the benzo ring thereof by the group R⁹ as defined hereunder or
(2) phenyl or phenyl mono- or disubstituted independently with halogen, hydroxy, cyano, carboxy, -O(C₁₋₄alkyl), -O(CH₂C₆H₅), -COO(C₁₋₄alkyl), amino, dimethylamino, -NHR¹⁰, 1-pyrrolidinyl or tetrazolyl; or
(3) pyridine or pyridinyl mono- or disubstituted independently with halogen, methyl, hydroxy, nitro, cyano, carboxy, -O(C₁₋₄ alkyl), -O(CH₂C₆H₅),-COO(C₁₋₄alkyl), amino or dimethylamino; or
(4) -NHR¹¹ where R¹¹ is defined hereinunder or R¹¹ is 7-indazolyl containing a group R¹⁰ at the N-1 position;
R³ is hydrogen, C₁₋₆alkyl, C₃₋₆cycloalkyl, phenyl or phenyl mono- or disubstituted independently with halogen;
R⁴ is independently C₃₋₆alkyl, C₃₋₆cycloalkyl, C₃₋₆alkenyl, phenyl, -(CH2)pCN or -(CH2)pCOO(C₁₋₄alkyl) and R⁵ is independently C₃₋₆alkyl, C₃₋₆cycloalkyl, C3-6 alkenyl, benzyl, phenyl or phenyl mono- or disubstituted independently with C₁₋₃alkyl, cyano, hydroxy, dimethylamino, -O(C₁₋₄alkyl), -O(CH₂C₆H₅),-NH(C₁₋₄alkyl), -COO(C₁₋₄alkyl), -N(C₁₋₄alkyl)₂ pyrrolidino, morpholino or halogen or R⁴ is C₁₋₂alkyl and R⁵ is phenyl substituted at the 2- or 4- position with chloro, methyl, methoxy or methoxycarbonyl;
R⁶ is hydrogen or methyl;
R⁷ is hydrogen, hydroxy, fluoro, dimethylamino, -O(C₁₋₄alkyl) or -O(CH₂C₆H₅);
R⁸ is -(CH₂)_{b}COOH;
R⁹ is methyl, chloro, nitro, hydroxy, methoxy or -NHR¹⁰;
R¹⁰ is hydrogen, acetyl, C₁₋₄alkyl, -SO₃H, -SO₂CH₃, -SO₂CF₃ or -SO₂C₆H₅, C₁₋₄alkoxycarbonyl;
R¹¹ is phenyl or phenyl mono- or disubstituted independently with fluorine, trifluoromethoxy, C₁₋₄alkylthio, -(CH₂)_{c}COOH, -(CH₂)_{c}COO(C₁₋₄alkyl), -(CH₂)_{c}SCH₃, -(CH₂)_{c}SOCH₃, -(CH₂)_{c}SO₂CH₃, -(CH₂)_{c}CONH₂, -SCH₂COOH, -CONH(SO₂CH₃), -CONH(SO₂CF₃), -(CH₂)_{c}N(C₁₋₄alkyl)₂, -(CH₂)_{c}NH(SO₂CF₃),-(CH₂)_{c}N(SO₂CF₃)(C₁₋₄alkyl), -(CH₂)_{c}SO₂NHCO(C₁₋₄alkyl), -(CH₂)_{c}SO₂N(C₁₋₄alkyl)CO(C₁₋₄alkyl), -(CH₂)_{c}CONHSO₂(C₁₋₄alkyl), -(CH₂)_{c}CON(C₁₋₄alkyl)SO₂(C₁₋₄alkyl), -(CH₂)_{c}OR¹² -(CH₂)_{c}NHR¹⁰ or phenyl monosubstituted with -(CH₂)_{c}(tetrazolyl), -(CH₂)_{c} (carboxamidotetrazolyl) or-(CH₂)_{c}(pyrrolidinyl) or R¹¹ is selected from pyridine or pyridinyl mono- or disubstituted independently with halogen, methyl, hydroxy, nitro, cyano, carboxy, -O(C₁₋₄ alkyl), amino, dimethylamino, -NHR¹⁰;
R¹² is hydrogen, C₁₋₆alkyl, C₃₋₆cycloalkyl, -CH₂C₆H₅, -CH₂COOH,-CH₂CONH₂, -CH₂CONH(C₁₋₄alkyl), -CH₂CON(C₁₋₄alkyl)₂ or 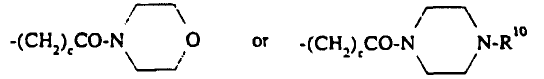
z is 1 or 2;
n is 1 or 2;
p is an integer from 1-4;
b is an integer from 0-3; and
c is 0 or 1;
together with one or more pharmaceutically acceptable carriers wherein the formulation encased in an enteric coating or capsule.

2. A pharmaceutical composition as claimed in Claim 1 wherein R¹ represents the group of Formula (II) wherein R⁶ is methyl, R⁷ is hydrogen, hydroxyl, methoxy or fluorine and n is 1 or R¹ represents the group NR⁴R⁵ wherein R⁴ represents C₃₋₆ alkyl, cyclohexyl or phenyl, and R⁵ represents C₃₋ ₆ alkyl or phenyl optionally substituted in the para position by hydroxy, dimethylamino, methoxy, fluorine, pyrrolidino or morpholino.

3. A pharmaceutical composition as claimed in Claims 1 or 2 wherein R¹ represents the group NR⁴R⁵ and R⁴ represents propyl or isopropyl and R⁵ represents phenyl or phenyl substituted in the para position by a group selected from hydroxy, methoxy, dimethylamino, fluorine, or morpholino.

4. A pharmaceutical composition as claimed in any of Claims 1 to 3 wherein R² represents a group selected from phenyl (optionally substituted by one or two groups which may be the same or different and selected from chlorine, fluorine, amino, hydroxy or carboxy,) or NHR¹¹ wherein R¹¹ is phenyl (optionally substituted by fluoro, hydroxy, amino, dimethylamino. trifluoromethylsulphonylamino, C₁₋₄ alkoxycarbonyl, carboxy, 1H-tetrazol-5-yl, acetylamino or OR¹² wherein R¹² represents hydrogen, methyl, benzyl, CH₂CO₂H, CH₂CONH₂, CH₂CONHCH₃, CH₂CON(CH₃)₂ 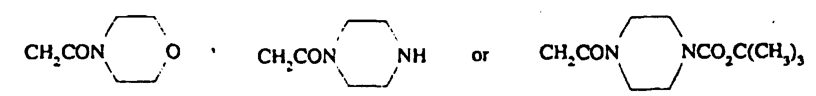 )or 7-indazolyl wherein the N-1 substituent R¹⁰ is hydrogen, or R² represents an indole group wherein the nitrogen atom is optionally substituted by the group-CH₂CO₂H and the benzo ring is optionally substituted by a group selected from chlorine, methyl, methoxy, nitro, hydroxy or amino.

5. A pharmaceutical composition as claimed in any of Claims 1-4 wherein R² represents an indole group which is unsubstituted on the nitrogen atom and in which the benzo ring thereof is optionally substituted by a group selected from chlorine, methyl, methoxy, nitro, hydroxy or amino.

6. A pharmaceutical composition as claimed in any of Claims 1-5 wherein R³ represents hydrogen, methyl, cyclohexyl,2-fluorophenyl or phenyl.

7. A pharmaceutical composition as claimed in any of Claims 1-6 wherein R³ represents phenyl.

8. A pharmaceutical composition as claimed in any of Claims 1-7 wherein X represents hydrogen.

9. A pharmaceutical composition as claimed in Claim 1 wherein R¹ represents NR⁴R⁵ and R⁴ represents isopropyl and R⁵ represents p-methoxyphenyl; R² represents an unsubstituted 2-indole group; R³ represents phenyl and X represents hydrogen and enantiomers thereof.

10. A pharmaceutical composition as claimed in claim 1 in the form of an enterically coated tablet.

## Patentansprüche

1. Pharmazeutische Formulierung in fester Arzneiform zur oralen Verabreichung, die eine Verbindung der Formel (I) 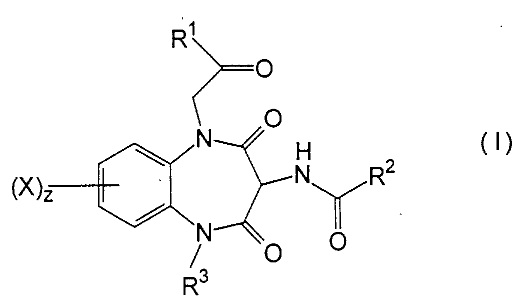 und physiologische Salze und Solvate davon umfasst, worin:
X entweder Wasserstoff, Trifluormethyl, Alkyl, C₁₋₄-Alkylthio, -O(C₁₋₄-Alkyl) oder Halogen ist;
R¹ entweder Formel (II) oder -NR⁴R⁵ ist; 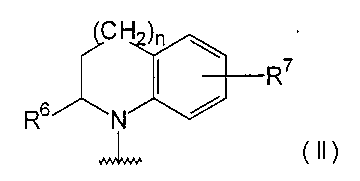
R² entweder:
(1) ein an seiner 2-Position gebundener Heterocyclus, der aus Pyrrol, Tetrahydropyrrol, Indol, Benzofuran, Thiophen, Benzothiophen, Indolin, Chinolin oder 4-Oxobenzopyran ausgewählt ist und worin das Pyrrol, Tetrahydropyrrol, Indol oder Indolin gegebenenfalls am Ringstickstoff davon mit der Gruppe R⁸ wie nachfolgend definiert substituiert sein kann und das Indol, Indolin, Chinolin, Benzofuran, Benzothiophen oder 4-Oxobenzopyran gegebenenfalls im Benzoring davon mit der Gruppe R⁹ wie nachfolgend definiert substituiert sein kann; oder
(2) Phenyl oder unabhängig mit Halogen, Hydroxy, Cyano, Carboxy, - (C₁₋₄-Alkyl), -O(CH₂C₆H₅), -COO(C₁₋₄-Alkyl), Amino, Dimethylamino, -NHR¹⁰, 1-Pyrrolidinyl oder Tetrazolyl mono- oder disubstituiertes Phenyl; oder
(3) Pyridin oder unabhängig mit Halogen, Methyl, Hydroxy, Nitro, Cyano, Carboxy, -O(C₁₋₄-Alkyl), -O(CH₂C₆H₅), -COO(C₁₋₄-Alkyl), Amino oder Dimethylamino mono- oder disubstituiertes Pyridinyl; oder
(4) -NHR¹¹ ist, worin R¹¹ wie nachfolgend definiert ist oder R¹¹ 7-Indazolyl ist, das eine Gruppe R¹⁰ an der N-1-Position enthält;
R³ Wasserstoff, C₁₋₆-Alkyl, C₃₋₆-Cycloalkyl, Phenyl oder unabhängig mit Halogen mono- oder disubstituiertes Phenyl ist;
R⁴ unabhängig C₃₋₆-Alkyl, C₃₋₆-Cycloalkyl, C₃₋₆-Alkenyl, Phenyl, -(CH₂)ₚCN oder -(CH₂)ₚCOO(C₁₋₄-Alkyl) ist und R⁵ unabhängig C₃₋₆-alkyl, C₃₋₆-Cycloalkyl, C₃₋₆-Alkenyl, Benzyl, Phenyl oder unabhängig mit C₁₋₃-Alkyl, Cyano, Hydroxy, Dimethylamino, -O(C₁₋₄-Alkyl), -O(CH₂C₆H₅), -NH(C₁₋₄-Alkyl), -COO(C₁₋₄-Alkyl), -N(C₁₋₄-Alkyl)₂, Pyrrolidino, Morpholino oder Halogen mono- oder disubstituiertes Phenyl ist oder R⁴ C₁₋₂-Alkyl ist und R⁵ in der 2- oder 4-Position mit Chlor, Methyl, Methoxy oder Methoxycarbonyl substituiertes Phenyl ist;
R⁶ Wasserstoff oder Methyl ist;
R⁷ Wasserstoff, Hydroxy, Fluor, Dimethylamino, -O(C₁₋₄-Alkyl) oder -O(CH₂C₆H₅) ist;
R⁸ -(CH₂)_{b}COOH ist;
R⁹ Methyl, Chlor, Nitro, Hydroxy, Methoxy oder -NHR¹⁰ ist;
R¹⁰ Wasserstoff, Acetyl, C₁₋₄-Alkyl, -SO₃H, -SO₂CH₃, -SO₂CF₃ oder -SO₂C₆H₅, C₁₋₄-Alkoxycarbonyl ist;
R¹¹ Phenyl oder unabhängig mit Fluor, Trifluormethoxy, C₁₋₄-Alkylthio, -(CH₂)_{c}COOH, -(CH₂)_{c}COO(C₁₋₄-Alkyl), -(CH₂)_{c}SCH₃, -(CH₂)_{c}SOCH₃, -(CH₂)_{c}SO₂CH₃, -(CH₂)_{c}CONH₂, -SCH₂COOH, -CONH(SO₂CH₃), -CONH(SO₂CF₃), -(CH₂)_{c}N(C₁₋₄-Alkyl)₂, -(CH₂)_{c}NH(SO₂CF₃), -(CH₂)_{c}N(SO₂CF₃)(C₁₋₄-Alkyl), -(CH₂)_{c}SO₂NHCO(C₁₋₄-Alkyl), -(CH₂)_{c}SO₂N(C₁₋₄-Alkyl)CO(C₁₋₄-alkyl), -(CH₂)_{c}CONHSO₂(C₁₋₄-Alkyl), -(CH₂)_{c}CON(C₁₋₄-Alkyl)SO₂(C₁₋₄-alkyl), -(CH₂)_{c}OR¹², -(CH₂)_{c}NHR¹⁰ mono- oder disubstituiertes Phenyl oder mit -(CH₂)_{c}(Tetrazolyl), -(CH₂)_{c}(Carboxamidotetrazolyl) oder -(CH₂)_{c}(Pyrrolidinyl) monosubstituiertes Phenyl ist oder R¹¹ aus Pyridin oder unabhängig mit Halogen, Methyl, Hydroxy, Nitro, Cyano, Carboxy, - O(C₁₋₄-Alkyl), Amino, Dimethylamino, -NHR¹⁰ mono- oder disubstituiertem Pyridinyl ausgewählt ist;
R¹² Wasserstoff, C₁₋₆-Alkyl, C₃₋₆-Cycloalkyl, -CH₂C₆H₅, -CH₂COOH, -CH₂CONH₂, -CH₂CONH(C₁₋₄-Alkyl), -CH₂CON(C₁₋₄-Alkyl)₂ oder 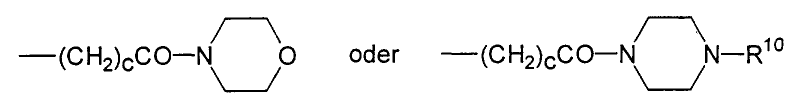 ist;
z 1 oder 2 ist;
n 1 oder 2 ist;
p eine ganze Zahl von 1 bis 4 ist;
b eine ganze Zahl von 0 bis 3 ist; und
c 0 oder 1 ist,
zusammen mit einem oder mehreren pharmazeutisch akzeptablen Trägern, worin die Formulierung in einem magensaftresistenten Überzug oder einer magensaftresistenten Kapsel verkapselt ist.

2. Pharmazeutische Zusammensetzung gemäss Anspruch 1, worin R¹ die Gruppe der Formel (II) darstellt, worin R⁶ Methyl ist, R⁷ Wasserstoff, Hydroxyl, Methoxy oder Fluor ist und n 1 ist, oder R¹ die Gruppe NR⁴R⁵ darstellt, worin R⁴ C₃₋₆-Alkyl, Cyclohexyl oder Phenyl darstellt, und R⁵ C₃₋₆-Alkyl oder Phenyl darstellt, das gegebenenfalls in der para-Position mit Hydroxy, Dimethylamino, Methoxy, Fluor, Pyrrolidino oder Morpholino substituiert ist.

3. Pharmazeutische Zusammensetzung gemäss Anspruch 1 oder 2, worin R¹ die Gruppe NR⁴R⁵ darstellt und R⁴ Propyl oder Isopropyl darstellt und R⁵ Phenyl oder in der para-Position mit einer Gruppe substituiertes Phenyl darstellt, die aus Hydroxy, Methoxy, Dimethylamino, Fluor oder Morpholino ausgewählt ist.

4. Pharmazeutische Zusammensetzung gemäss einem der Ansprüche 1 bis 3, worin R² eine Gruppe darstellt, ausgewählt aus Phenyl (gegebenenfalls substituiert mit einer oder zwei Gruppen, die gleich oder verschieden sein können und ausgewählt sind aus Chlor, Fluor, Amino, Hydroxy oder Carboxy) oder NHR¹¹, worin R¹¹ Phenyl (gegebenenfalls substituiert mit Fluor, Hydroxy, Amino, Dimethylamino, Trifluormethylsulfonylamino, C₁₋₄-Alkoxycarbonyl, Carboxy, 1H-Tetrazol-5-yl, Acetylamino oder OR¹² ist, worin R¹² Wasserstoff, Methyl, Benzyl, CH₂CO₂H, CH₂CONH₂, CH₂CONHCH₃, CH₂CON(CH₃)₂ 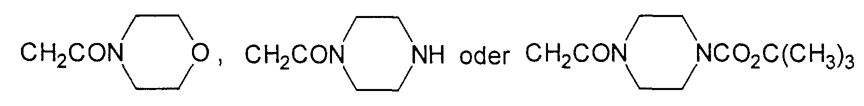 ist) oder 7-Indazolyl ist, worin der N-1-Substituent R¹⁰ Wasserstoff ist oder R² eine Indolgruppe darstellt, worin das Stickstoffatom gegebenenfalls mit der Gruppe -CH₂CO₂H substituiert ist und der Benzoring gegebenenfalls mit einer Gruppe substituiert ist, die aus Chlor, Methyl, Methoxy, Nitro, Hydroxy oder Amino ausgewählt ist.

5. Pharmazeutische Zusammensetzung gemäss einem der Ansprüche 1 bis 4, worin R² eine Indolgruppe darstellt, die am Stickstoffatom unsubstituiert ist und worin der Benzoring davon gegebenenfalls mit einer Gruppe substituiert ist, die aus Chlor, Methyl, Methoxy, Nitro, Hydroxy oder Amino ausgewählt ist.

6. Pharmazeutische Zusammensetzung gemäss einem der Ansprüche 1 bis 5, worin R³ Wasserstoff, Methyl, Cyclohexyl, 2-Fluorphenyl oder Phenyl darstellt.

7. Pharmazeutische Zusammensetzung gemäss einem der Ansprüche 1 bis 6, worin R³ Phenyl darstellt.

8. Pharmazeutische Zusammensetzung gemäss einem der Ansprüche 1 bis 7, worin X Wasserstoff darstellt.

9. Pharmazeutische Zusammensetzung gemäss Anspruch 1, worin R¹ NR⁴R⁵ darstellt und R⁴ Isopropyl darstellt und R⁵ p-Methoxyphenyl darstellt; R² eine unsubstituierte 2-Indolgruppe darstellt; R³ Phenyl darstellt und X Wasserstoff darstellt und Enantiomere davon.

10. Pharmazeutische Zusammensetzung gemäss Anspruch 1 in Form einer magensaftresistent überzogenen Tablette.

## Revendications

1. Formulation pharmaceutique sous une forme de dosage solide pour une administration orale, qui comprend un composé de formule (I) 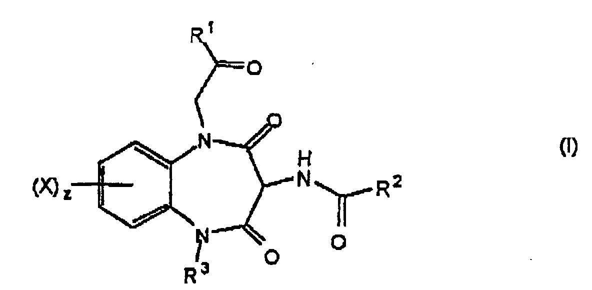 et des sels et des solvates physiologiques de ceux-ci, dans laquelle :
X est soit un atome d'hydrogène, un groupe trifluorométhyle, un groupe alkyle, un groupe (alkyle en C₁ à C₄)thio, un groupe -O(alkyle en C₁ à C₄) ou un atome d'halogène ;
R¹ est soit une formule II ou un groupe -NR⁴R⁵ ; 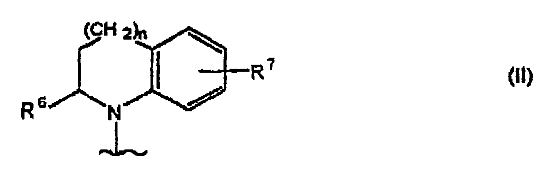
R² est soit :
(1) un hétérocycle lié au niveau de sa 2ème position et choisi parmi un hétérocycle pyrrole, un hétérocycle tétrahydropyrrole, un hétérocycle indole, un hétérocycle benzofuranne, un hétérocycle thiophène, un hétérocycle benzothiophène, un hétérocycle indoline, un hétérocycle quinoléine ou un hétérocycle 4-oxobenzopyranne et dans lequel ledit hétérocycle pyrrole, ledit hétérocycle tétrahydropyrrole, ledit hétérocycle indole, ledit hétérocycle indoline peuvent être éventuellement substitués sur l'atome d'azote de l'anneau par le groupe R⁸ tel que défini ci-dessous et ledit hétérocycle indole, ledit hétérocycle indoline, ledit hétérocycle quinoléine, ledit hétérocycle benzofuranne, ledit hétérocycle benzothiophène ou ledit hétérocycle 4-oxobenzopyranne peut éventuellement être substitué dans l'anneau benzo par le groupe R⁹ tel que défini ci-dessous ou
(2) un groupe phényle ou un groupe phényle monosubstitué ou disubstitué, indépendamment, par un atome d'halogène, un groupe hydroxy, un groupe cyano, un groupe carboxy, un groupe -O(alkyle en C₁ à C₄), un groupe -O(CH₂C₆H₅), un groupe -COO(alkyle en C₁ à C₄), un groupe amino, un groupe diméthylamino, un groupe -NHR¹⁰, un groupe 1-pyrrolidinyle ou un groupe tétrazolyle ; ou
(3) un groupe pyridine ou un groupe pyridinyle monosubstitué ou disubstitué, indépendamment, par un atome d'halogène, un groupe méthyle, un groupe hydroxy, un groupe nitro, un groupe cyano, un groupe carboxy, un groupe -O(alkyle en C₁ à C₄), un groupe -O(CH₂C₆H₅), un groupe -COO(alkyle en C₁ à C₄), un groupe amino ou un groupe diméthylamino ; ou
(4) un groupe -NHR¹¹, où R¹¹ est défini ci-dessous ou R¹¹ est un groupe 7-indazolyle contenant un groupe R¹⁰ en position N-1 ;
R³ représente un atome d'hydrogène, un groupe alkyle en C₁ à C₆, un groupe cycloalkyle en C₃ à C₆, un groupe phényle ou un groupe phényle monosubstitué ou disubstitué, indépendamment, par un atome d'halogène ;
R⁴ représente, indépendamment, un groupe alkyle en C₃ à C₆, un groupe cycloalkyle en C₃ à C₆, un groupe alcényle en C₃ à C₆, un groupe phényle, un groupe -(CH₂)ₚCN ou un groupe -(CH₂)ₚCOO(alkyle en C₁ à C₄) et R⁵ est, indépendamment, un groupe alkyle en C₃ à C₆, un groupe cycloalkyle en C₃ à C₆, un groupe alcényle en C₃ à C₆, un groupe benzyle, un groupe phényle ou un groupe phényle monosubstitué ou disubstitué, indépendamment, par un groupe alkyle en C₁ à C₃, un groupe cyano, un groupe hydroxy, un groupe diméthylamino, un groupe -O(alkyle en C₁ à C₄), un groupe -O(CH₂C₆H₅), un groupe NH(alkyle en C₁ à C₄), un groupe -COO(alkyle en C₁ à C₄), un groupe -N(alkyle en C₁ à C₄)₂, un groupe pyrrolidino, un groupe morpholino ou un atome d'halogène ou R⁴ est un groupe alkyle en C₁ à C₂ et R⁵ est un groupe phényle substitué en 2ème ou en 4ème position par un groupe chloro, un groupe méthyle, un groupe méthoxy ou un groupe méthoxycarbonyle ;
R⁶ représente un atome d'hydrogène ou un groupe méthyle ;
R⁷ représente un atome d'hydrogène, un groupe hydroxy, un groupe fluoro, un groupe diméthylamino, un groupe -O(alkyle en C₁ à C₄) ou un groupe -O(CH₂C₆H₅) ;
R⁸ représente un groupe -(CH₂)_{b}COOH ;
R⁹ représente un groupe méthyle, un groupe chloro, un groupe nitro, un groupe hydroxy, un groupe méthoxy ou un groupe -NHR¹⁰ ;
R¹⁰ représente un atome d'hydrogène, un groupe acétyle, un groupe alkyle en C₁ à C₄, un groupe -SO₃H, un groupe -SO₂CH₃, un groupe -SO₂CF₃ ou un groupe -SO₂C₆H₅, un groupe (alcoxy en C₁ à C₄)carbonyle ;
R¹¹ représente un groupe phényle ou un groupe phényle monosubstitué ou disubstitué, indépendamment, par un atome de fluor, un groupe trifluorométhoxy, un groupe (alkyle en C₁ à C₄)thio, un groupe -(CH₂)_{c}COOH, un groupe -(CH₂)_{c}COO(alkyle en C₁ à C₄), un groupe -(CH₂)_{c}SCH₃, un groupe -(CH₂)_{c}SOCH₃, un groupe -(CH₂)_{c}SO₂CH₃, un groupe -(CH₂)_{c}CONH₂, un groupe -SCH₂COOH, un groupe -CONH(SO₂CH₃), un groupe -CONH(SO₂CF₃), un groupe -(CH₂)_{c}N(alkyle en C₁ à C₄)₂, un groupe -(CH₂)_{c}NH(SO₂CF₃), un groupe -(CH₂)_{c}N(SO₂CF₃)(alkyle en C₁ à C₄), un groupe -(CH₂)_{c}SO₂NHCO(alkyle en C₁ à C₄), un groupe -(CH₂)_{c}SO₂N(alkyle en C₁ à C₄)CO(alkyle en C₁ à C₄), un groupe -(CH₂)_{c}CONHSO₂(alkyle en C₁ à C₄), un groupe -(CH₂)_{c}CON(alkyle en C₁ à C₄)SO₂(alkyle en C₁ à C₄), un groupe -(CH₂)_{c}OR¹²-(CH₂)_{c}NHR¹⁰ ou un groupe phényle monosubstitué par un groupe -(CH₂)_{c}(tétrazolyle), un groupe -(CH₂)_{c}(carboxamidotétrazolyle) ou un groupe -(CH₂)_{c}(pyrrolidinyle), ou R¹¹ est choisi parmi un groupe pyridine ou un groupe pyridinyle monosubstitué ou disubstitué, indépendamment, par un atome d'halogène, un groupe méthyle, un groupe hydroxy, un groupe nitro, un groupe cyano, un groupe carboxy, un groupe -O(alkyle en C₁ à C₄), un groupe amino, un groupe diméthylamino, un groupe -NHR¹⁰ ;
R¹² représente un atome d'hydrogène, un groupe alkyle en C₁ à C₆, un groupe cycloalkyle en C₃ à C₆, un groupe -CH₂C₆H₅, un groupe -CH₂COOH, un groupe -CH₂CONH₂, un groupe -CH₂CONH(alkyle en C₁ à C₄), un groupe -CH₂CON(alkyle en C₁ à C₄)₂ ou 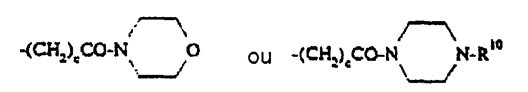
z est égal à 1 ou 2 ;
n est égal à 1 ou 2 ;
p est un nombre entier de 1 à 4 ;
b est un nombre entier de 0 à 3 et
c est égal à 0 ou 1,
avec un ou plusieurs supports pharmaceutiquement acceptables, dans laquelle la formulation est enveloppée dans un revêtement ou une capsule entérique.

2. Composition pharmaceutique telle que revendiquée dans la formule 1, dans laquelle R¹ représente un groupe de formule (II) dans laquelle R⁶ représente un groupe méthyle, R⁷ représente un atome d'hydrogène, un groupe hydroxyle, un groupe méthoxy ou un atome de fluor et n est égal à 1 ou R¹ représente le groupe NR⁴R⁵, dans lequel R⁴ représente un groupe alkyle en C₃ à C₆, un groupe cyclohexyle ou un groupe phényle et R⁵ représente un groupe alkyle en C₃ à C₆ ou un groupe phényle, éventuellement substitué en position para par un groupe hydroxy, un groupe diméthylamino, un groupe méthoxy, un atome de fluor, un groupe pyrrolidino ou un groupe morpholino.

3. Composition pharmaceutique telle que revendiquée dans les revendications 1 ou 2, dans laquelle R¹ représente le groupe NR⁴R⁵ et R⁴ représente un groupe propyle ou un groupe isopropyle et R⁵ représente un groupe phényle ou un groupe phényle substitué en position para par un groupe choisi parmi un groupe hydroxy, un groupe méthoxy, un groupe diméthylamino, un atome de fluor ou un groupe morpholino.

4. Composition pharmaceutique telle que revendiquée dans les revendications 1 à 3, dans laquelle R² représente un groupe choisi parmi un groupe phényle (éventuellement substitué par un ou deux groupes qui peuvent être identiques ou différents et choisis parmi un atome de chlore, un atome de fluor, un groupe amino, un groupe hydroxy ou un groupe carboxy) ou un groupe NHR¹¹, dans lequel R¹¹ est un groupe phényle (éventuellement substitué par un groupe fluoro, un groupe hydroxy, un groupe amino, un groupe diméthylamino, un groupe trifluorométhylsulfonylamino, un groupe (alcoxy en C₁ à C₄)carbonyle, un groupe carboxy, un groupe 1H-tétrazol-5-yle, un groupe acétylamino, ou un groupe OR¹² dans lequel R¹² représente un atome d'hydrogène, un groupe méthyle, un groupe benzyle, un groupe CH₂CO₂H, un groupe CH₂CONH₂, un groupe CH₂CONHCH₃, un groupe CH₂CON(CH₃)₂, 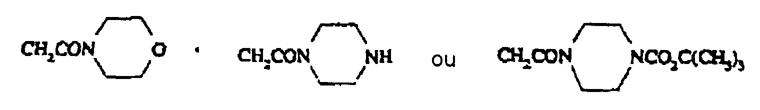 ) ou un groupe 7-indazolyle dans lequel le substituant N-1 R¹⁰ est un atome d'hydrogène, ou R² représente un groupe indole dans lequel l'atome d'azote est éventuellement substitué par un groupe CH₂CO₂H et l'anneau benzo est éventuellement substitué par un groupe choisi parmi un atome de chlore, un groupe méthyle, un groupe méthoxy, un groupe nitro, un groupe hydroxy ou un groupe amino.

5. Composition pharmaceutique telle que revendiquée dans l'une quelconque des revendications 1 à 4, dans laquelle R² représente un groupe indole qui n'est pas substitué sur l'atome d'azote et dans laquelle l'anneau benzo est éventuellement substitué par un groupe choisi parmi un atome de chlore, un groupe méthyle, un groupe méthoxy, un groupe nitro, un groupe hydroxy ou un groupe amino.

6. Composition pharmaceutique telle que revendiquée dans l'une quelconque des revendications 1 à 5, dans laquelle R³ représente un atome d'hydrogène, un groupe méthyle, un groupe cyclohexyle, un groupe 2-fluorophényle ou un groupe phényle.

7. Composition pharmaceutique telle que revendiquée dans l'une quelconque des revendications 1 à 6, dans laquelle R³ représente un groupe phényle.

8. Composition pharmaceutique telle que revendiquée dans l'une quelconque des revendications 1 à 7, dans laquelle X représente de l'hydrogène.

9. Composition pharmaceutique telle que revendiquée dans la revendication 1, dans laquelle R¹ représente un groupe NR⁴R⁵ et R⁴ représente un groupe isopropyle et R⁵ représente un groupe p-méthoxyphényle ; R² représente un groupe indole non substitué ; R³ représente un groupe phényle et X représente de l'hydrogène et des énantiomères de celle-ci.

10. Composition pharmaceutique telle que revendiquée dans la revendication 1 sous forme d'un cachet revêtu entériquement.
